(19) Europäisches Patentamt
European Patent Office
Office européen des brevets

(11) Numéro de publication: **0 262 993 B1**

(12) **FASCICULE DE BREVET EUROPEEN**

(45) Date de publication du fascicule du brevet: **12.09.90**

(51) Int. Cl.⁵: **C07D 241/08**, C07D 403/12, C07D 401/06, C07D 403/14, C07D 401/14, A61K 31/495

(21) Numéro de dépôt: **87401613.2**

(22) Date de dépôt: **09.07.87**

(54) **Nouveaux dérivés de la pipérazine dione-2,6, leurs procédés de préparation et les compositions pharmaceutiques qui les contiennent.**

(30) Priorité: **09.07.86 FR 8609977**

(43) Date de publication de la demande: **06.04.88 Bulletin 88/14**

(45) Mention de la délivrance du brevet: **12.09.90 Bulletin 90/37**

(84) Etats contractants désignés: **AT BE CH DE FR GB IT LI LU NL SE**

(56) Documents cités:
**EP-A- 0 125 475**
**DE-A- 2 511 891**
**FR-A- 2 013 813**

(73) Titulaire: **ADIR ET COMPAGNIE, 22, rue Garnier, F-92201 Neuilly sur Seine(FR)**

(72) Inventeur: **Lavielle, Gilbert, 1 Avenue Lilly, F-78170 La Celle Saint-Cloud(FR)**
Inventeur: **Poignant, Jean-Claude, La Guyonnerie 13 rue de la Fontaine Saint Mathieu, F-91440 Bures Sur Yvette(FR)**

**EP 0 262 993 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux dérivés de la pipérazine dione-2,6, leurs procédés de préparation et les compositions pharmaceutiques les renfermant.

On connait certains dérivés de la pipérazine dione-2,6 ayant des effets sédatifs sur le système nerveux central. (De Jong D. et coll. J. Pharm. 1959, 11, p. 393-399).

Les nouveaux dérivés de la pipérazine dione-2,6 possèdent des propriétés pharmacologiques très intéressantes et notamment des propriétés anxiolytiques antiagressives et antipsychotiques très puissantes. En revanche, ils sont dépourvus des effets secondaires classiquement rencontrés dans cette classe pharmacologique. En effet, les composés de la présente invention n'ont pas d'effets sédatifs, anticonvulsivants ou myorelaxants et se distinguent par ce fait d'autres dérivés de la pipérazine dione-2,6 déjà connus. D'autre part, les composés de l'invention par leur structure chimique se démarquent nettement des autres pipérazines déjà décrites.

La présente invention a plus particulièrement pour objet les dérivés de la pipérazine dione-2,6, de formule générale I:

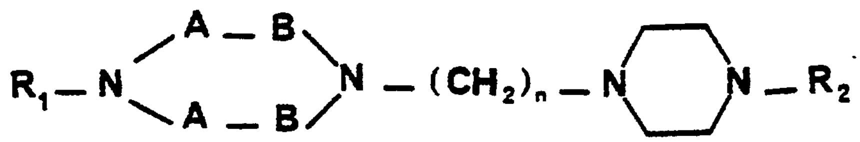

(I)

dans laquelle:

- A et B représentent chacun un radical méthylène ou un radical carbonyle, à condition toutefois que A et B ne représentent jamais simultanément le même radical,
- $R_1$ représente un radical diphénylméthyle éventuellement substitué sur les cycles benzèniques par un atome d'halogène, un radical cyclohexyle, un groupe pyridylméthyle, ou un radical benzyle éventuellement substitué par un atome d'halogène ou par un radical alcoxy renfermant de 1 à 4 atomes de carbone,
- $R_2$ représente un radical pyrimidinyle ou un radical phényle éventuellement substitué par un atome d'halogène, par un radical alkyle de 1 à 4 atomes de carbone, par un radical trifluorométhyle ou par un radical alcoxy renfermant de 1 à 4 atomes de carbone,
- n est un nombre entier pouvant prendre les valeurs de 2 à 4,

et leurs sels formés par addition à un acide minéral ou organique pharmaceutiquement acceptable.

La présente invention à également pour objet le procédé de préparation de composés de formule générale I, caractérisé en ce que :

- soit :

l'on condense une imide de formule générale II:

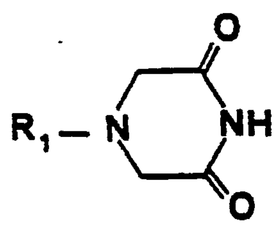

(II)

dans laquelle la définition du substituant $R_1$ demeure celle mentionnée précédemment, avec un composé de formule générale III :

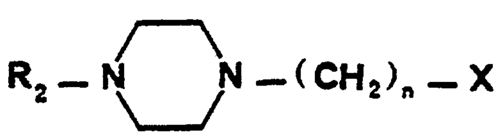

(III)

dans laquelle $R_2$ est tel que précédemment défini, n est un nombre entier égal à 2 ou 3 et X représente un atome de chlore ou de brome,

pour obtenir les composés de formule I dans laquelle la définition de $R_1$ et $R_2$ demeure celle indiquée précédemment, n est un nombre entier égal à 2 ou 3, A représente un méthylène et B un carbonyle,

ou

l'on fait réagir sur le composé de formule générale II le bromo-1 chloro-3 propane en présence d'un hydrure métallique pour obtenir un composé de formule générale IV :

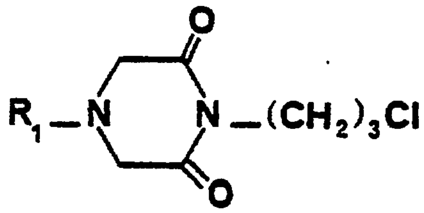

(IV)

dans laquelle $R_1$ a la signification précédemment définie pour la formule I,
et ensuite l'on condense la (chloro-3 propyl)-1 pipérazine dione-2,6 ainsi obtenue avec une pipérazine de formule générale V :

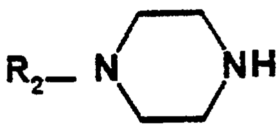

(V)

dans laquelle la définition de $R_2$ demeure celle indiquée précédemment, pour obtenir un composé de formule générale I dans laquelle $R_1$ et $R_2$ ont les significations précédemment définies, n est égal à 3, A représente un méthylène et B un carbonyle,
- <u>soit</u> :
l'on condense une morpholine dione-2,6 de formule générale VI :

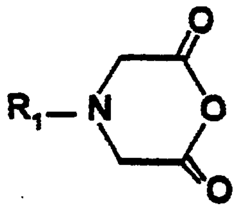

(VI)

dans laquelle $R_1$ a la signification précédemment définie pour la formule I, avec une pipérazinyl-4 butylamine de formule générale VII :

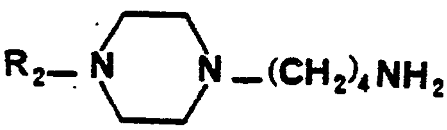

(VII)

dans laquelle la définition de $R_2$ reste identique à celle donnée pour la formule I,
pour obtenir un composé de formule I dans laquelle la définition de $R_1$ et $R_2$ demeure celle indiquée précédement, n est égal à 4, A représente un méthylène et B un carbonyle,
- <u>soit</u> :
l'on condense une pipérazine dione de formule générale VIII :

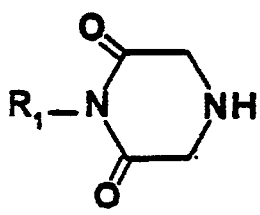

(VIII)

dans laquelle $R_1$ a la signification précédemment définie pour la formule I, avec un dérivé de pipérazine de formule générale III, pour obtenir un composé de formule générale I dans laquelle $R_1$, $R_2$ et n ont les significations précédemment définies, A représente un carbonyle et B représente un méthylène.

Les composés de formule générale I peuvent être ensuite transformés, si on le désire, en leurs sels d'addition à un acide minéral ou organique pharmaceutiquement acceptable.

Les imides de formule générale II sont obtenues par chauffage des diacides correspondants en présence de formamide. Ces derniers sont préparés par action de l'acide chloro-2 acétique sur les amines primaires correspondantes (Organic Synthese Collective ; John Wiley and Sons Ed. N.Y. 1943, Vol. II

p. 397). Elles peuvent être aussi synthétisées par alkylation de la pipérazine dione-2,6 par un halogènure d'alcane, (Bull. Soc. Chim. France, 1968, 8, 3248).

Les composés de formule générale III sont obtenus dans le cas où n est égal à 2 par condensation de l'oxyde d'éthylène sur la pipérazine correspondante (J.A.C.S. 1948, 70, p. 2015) et ensuite par chloration de l'alcool obtenu à l'aide du chlorure de thionyle. Dans le cas où n est égal à 3, les composés de formule générale III sont obtenus par action du bromo-1 chloro-3 propane sur la pipérazine correspondante. (Bull. Soc. Chim. France, 1968, 8, p. 3247).

La synthèse des morpholine diones-2,6 de formule générale VI est connue. (Organic Synthese Collective John Wiley and Sons Ed. N.Y. 1943, Vol. I p. 91).

Les pipérazinyl-4 butylamines de formule générale VII sont obtenues par réduction des nitriles correspondants en présence d'hydrures métalliques tels que l'hydrure double de lithium et d'aluminium. (J. Med. Chem., 1972, 15, 5, p. 477).

La préparation de ces nitriles est connue (Bull. Soc. Chim. France, 1968, 8, 3247).

Les composés de la formule générale VIII sont préparés par condensation de la benzyl-4 pipérazine dione-2,6 avec les halogénures d'alkyle correspondants, suivie d'une débenzylation par hydrogénolyse.

La condensation des imides de formule générale II avec les composés de formule générale III s'effectue de préférence dans un solvant organique anhydre polaire tel que le diméthylformamide en présence d'un hydrure métallique tel que l'hydrure de sodium et d'un sel minéral tel que l'iodure de sodium et à une température comprise entre 40°C et 100°C.

La condensation des pipérazine diones-2,6 de formule générale IV avec des pipérazines de formule générale V et celle des piperazine diones de formule générale VIII avec le composés de formule générale III sont réalisées dans un solvant organique polaire tel que la butanone-2 en présence des sels minéraux tels que le carbonate de sodium et l'iodure de sodium à une température comprise entre 40° et 100°C.

La réaction des morpholine diones-2,6 de formule générale VI avec les pipérazinyl-4 butylamines de formule générale VII s'effectue dans un solvant organique basique tel que la pyridine à une température entre 40°C et 100°C.

Parmi les acides pharmaceutiquement acceptables pour la préparation des sels d'addition aux composés de formule générale I, on peut citer les acides phosphorique, chlorhydrique, citrique, oxalique, sulfurique, tartrique, mandélique, triméthanesulfonique, etc...

Les composés selon l'invention, ainsi que leurs sels sont doués de propriétés pharmacologiques fort intéressantes.

En effet, les essais pharmacologiques in vivo ont montré que ces composés possèdent de puissantes propriétés anxiolytiques, antiagressives et antipsychotiques. Ces propriétés ont été mises en évidence au moyen d'essais classiquement utilisés chez l'animal permettant de présager avec une très bonne précision l'activité anxiolytique, antiagressive ou antipsychotique des nouveaux composés chez l'homme. (Dallas Treit, Neur. Biob. Rev., 1985, 9,p. 203-222).

En revanche, les composés de la présente invention sont dépourvus de propriétés sédatives du système nerveux central et se distinguent d'autres dérivés de la pipérazine dione-2,6 déjà connus.

Les composés de la présente invention présentent en particulir un profil d'agent psychotrope anxiosélectif. Leurs propriétés pharmacologiques permettent leur application dans le traitement de l'anxiété sous toutes ses formes.

L'invention s'étend aussi aux compositions pharmaceutiques renfermant comme principe actif au moins un composé de formule générale I ou l'un de ses sels d'addition avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, glossettes ou autres préparations galéniques appropriées pour une administration sublinguale, suppositoires, solutions injectables ou buvables.

La posologie peut varier largement en fonction de l'âge du poids du patient, de la nature et de la sévérité de l'affection ainsi que de la voie d'administration.

La voie d'administration préférée est la voie orale ou parentérale. D'une manière générale, la posologie unitaire s'échelonnera entre 0,1 et 100 mg et la posologie journalière, utilisable en thérapeutique humaine entre 0,1 et 300 mg.

Les exemples suivants, donnés à titre non-limitatif, illustrent l'invention.

Les points de fusion indiqués sont mesurés selon la technique micro-Köfler. Les spectres infra-rouge sont obtenus avec des solutions des produits dans le Nujol. Les spectres de résonnance magnétique nucléaire du proton (R.M.N.) ont été enregistrés à 60 MHz.

EXEMPLE 1 :

Dimandélate de la (fluoro-2 benzyl)-4 [((trifluorométhyl-3 phényl)-1 pipérazinyl-4)-3 propyl]-1 pipérazine dione-2,6

STADE A :

(chloro-3 propyl)-1 (fluoro-2 benzyl)-4 pipérazine dione-2,6

Préparer une suspension de 0,12 mole d'hydrure de sodium dans 100 ml de diméthylformamide. Ajouter 0,12 mole de (fluoro-2 benzyl)-4 pipérazine dione-2,6 en solution dans 50 ml de diméthylformamide. Chauffer à 70°C pendant 30 mn. Refroidir et ajouter 0,13 mole de bromo-1 chloro-3 propane. Laisser réagir à la température ambiante jusqu'à disparition complète de l'imide. Eliminer ensuite le solvant organique sous vide et reprendre le résidu par 100 ml d'eau et 200 ml de benzène.

Décanter, éliminer la phase aqueuse et évaporer la phase organique. Le résidu est trituré dans l'éther. On obtient des cristaux purs de la (chloro-3 propyl)-1 (fluoro-2 benzyl)-4 pipérazine dione-2,6.
Rendement : 96 %.

STADE B :

Ajouter dans une solution de 0,025 mole de (chloro-3 propyl)-1 (fluoro-2 benzyl)-4 pipérazine dione-2,6 précédemment obtenue et de 0,0275 mole de (trifluorométhyl-3 phényl)-1 pipérazine dans 200 ml de butanone-2, un mélange contenant 6 g de carbonate de sodium et 0,5 g d'iodure de sodium. Porter au reflux pendant 40 heures et ensuite filtrer et concentrer sous vide. Reprendre le résidu dans du benzène et laver la phase benzénique trois fois à l'eau distillée. Sécher la phase organique sur sulfate de sodium anhydride et évaporer le solvant. Purifier le résidu obtenu par chromatographie sur colonne de silice en utilisant comme éluant un mélange de dichlorométhane et de méthanol (95/5). Evaporer l'éluant et dissoudre le résidu dans le minimum d'acétone. Ajouter sous agitation 0,028 mole d'acide mandélique. Précipiter le dimandélate de la (fluoro-2 benzyl)-4 [((trifluorométhyl-3 phényl)-1 pipérazinyl-4)-3 propyl]-1 pipérazine dione-2,6 en ajoutant un mélange d'éther isopropylique et d'hexane (50/50). Rendement : 54 %
Point de fusion : 82°C

Les constantes physiques spectrales de la base sont indiquées dans le tableau I.

EXEMPLE 2-4 :

Les composés suivants ont été préparés selon le procédé décrit dans l'exemple 1. Leurs stuctures physiques spectrales sont indiquées dans le tableau I.

EXEMPLE 2 :

Dimandélate de la (fluoro-2 benzyl)-4 [((chloro-3 phényl)-1 pipérazinyl-4)-3 propyl]-1 pipérazine dione-2,6

Rendement 60 %
Point de fusion : 50°C

EXEMPLE 3 :

Dimandélate de la (chloro-2 benzyl)-4 [((chloro-3 phényl)-1 pipérazinyl-4)-3 propyl]-1 pipérazine dione-2,6

Rendement : 44 %
Point de fusion : 75°C

EXEMPLE 4 :

Dimandélate de la (chloro-2 benzyl)-4 [((trifluorométhyl-3 phényl)-1 pipérazinyl-4)-3 propyl]-1 pipérazine dione-2,6

Rendement : 68 %
Point de fusion : 92°C.

EXEMPLE 5 :

Trichlorhydrate de la benzyl-4 [((pyrimidinyl-2)-1 pipérazinyl-4)-4 butyl]-1 pipérazine dione-2,6

Chauffer à reflux pendant 12 heures 0,066 mole de N-benzyl morpholine dione-2,6 et 0,066 mole de (pyrimidinyl-2)-4 (amino-4 butyl)-1 pipérazine en solution dans 200 ml de pyridine.

Evaporer ensuite le solvant organique sous vide et chromatographier le résidu obtenu sur 500 g de silice fine en utilisant comme éluant un mélange de dichlorométhane et de méthanol (95/5). Evaporer l'éluant sous vide et dissoudre le résidu obtenu dans le minimum d'acétone. Ajouter sous agitation 12,6 ml d'étha-

nol chlorhydrique 5,28 N et isoler le sel précipité par filtration.
Rendement : 35 %
Point de fusion : 175°C.

Les constantes physiques spectrales du trichlorhydrate de la benzyl-4 [((pyrimidinyl-2)-1 pipérazinyl-4)-4 butyl]-1 pipérazine dione-2,6 sont indiquées dans le tableau I.

EXEMPLE 6 :

Dichlorhydrate de la (méthoxy-2 benzyl)-4 [((trifluorométhyl-3 phényl)-1 pipérazinyl-4)-4 butyl]-1 pipérazine dione-2,6

Chauffer à reflux pendant 40 heures, 0.022 mole de (méthoxy-2 benzyl)-4 morpholine dione-2,6 et 0,022 mole de (trifluorométhyl-3 benzyl)-4 (amino-4 butyl)-1 pipérazine dans 80 ml de pyridine. Concentrer ensuite le milieu réactionnel sous vide et laver à l'aide d'une solution saturée de carbonate de sodium. Extraire par le chloroforme, sécher sur sulfate de sodium anhydre et évaporer la phase chloroformique sous vide. Chromatographier le résidu sur une colonne contenant 350 g de silice fine en utilisant comme éluant un mélange de dichlorométhane et de méthanol (95/5). Récupérer l'éluat, évaporer sous vide et dissoudre le résidu dans l'éther éthylique contenant de l'acétone. Ajouter dans cette solution sous agitation, 8,3 ml d'éthanol chlorhydrique 5,28 N et isoler le sel formé par filtration.
Rendement : 64 %.
Point de fusion : 120°C.

Les constantes physiques spectrales de la base correspondante sont indiquées dans le tableau I.

EXEMPLE 7 :

Trichlorhydrate de la (pyridyl-2 méthyl)-4 [((fluoro-4 phényl)-1 pipérazinyl-4)-3 propyl]-1 pipérazine dione-2,6

Chauffer à 60°C une suspension de 0,098 mole d'hydrure de sodium dans 30 ml de diméthylformamide anhydre. Ajouter goutte à goutte, une solution de 0,098 mole de (pyridyl-2 méthyl)-4 pipérazine dione-2,6 dans 15 ml de diméthylformamide anhydre. L'addition terminée, maintenir le mélange à 60°C pendant deux heures. Refroidir ensuite le milieu réactionnel à 25°C et ajouter rapidement une solution de 0,126 mole de (fluoro-4 phényl)-4 (chloro-3 propyl)-1 pipérazine dans 10 ml de diméthylformamide anhydre et 0,1 g d'iodure de sodium. Chauffer à 70°C jusqu'à complète disparition de la pipérazine dione-2,6 de départ. Concentrer le milieu réactionnel sous vide. Recristalliser le résidu solide dans un mélange d'éther isopropylique et d'éthanol (90/10). On obtient des cristaux purs de la (pyridyl-2 méthyl)-4 [((fluoro-4 phényl)-1 pipérazinyl-4)-3 propyl]-1 pipérazine dione-2,6.
Rendement : 60 %
Point de fusion : 90°C

Ajouter à 0,0588 mole de la base obtenue précédemment 11,2 ml d'éthanol chlorhydrique 5,28 N, sous agitation. Isoler les cristaux du trichlorhydrate de la (pyridyl-2 méthyl)-4 [((fluoro-4 phényl)-1 pipérazinyl-4)-3 propyl]-1 pipérazine dione-2,6 par filtration.
Point de fusion : 120°C.

Les constantes physiques spectrales du trichlorhydrate de la (pyridyl-2 méthyl)-4 [((fluoro-4 phényl)-1 pipérazinyl-4)-3 propyl)-1 pipérazine dione-2,6 sont indiquées dans le tableau I.

Les composés suivants ont été préparés selon le procédé décrit dans l'exemple 7. Leurs constantes physiques spectrales sont indiquées dans le tableau I.

EXEMPLE 8 :

Chlorhydrate de la benzyl-4 [((chloro-3 phényl)-1 pipérazinyl-4)-3 propyl]-1 pipérazine dione-2,6

Rendement : 39 %
Point de fusion : 197°C.

EXEMPLE 9 :

Oxalate de la benzyl-4 [((fluoro-4 phényl)-1 pipérazinyl-4)-3 propyl]-1 pipérazine dione-2,6

Rendement : 45 %
Point de fusion : 190°C.

EXEMPLE 10 :

Dichlorhydrate de la benzyl-4 [((trifluorométhyl-3 phényl)-1 pipérazinyl-4)-2 ethyl]-1 pipérazine dione-2,6

Rendement : 38 %
Point de fusion : 196°C.

EXEMPLE 11 :

Dichlorhydrate de la (méthoxy-2 benzyl)-4 [((chloro-3 phényl)-1 pipérazinyl-4)-3 propyl]-1 pipérazine dione-2,6

Rendement : 50 %
Point de fusion : 150°C.

EXEMPLE 12 :

Dichlorhydrate de la (méthoxy-2 benzyl)-4 [((fluoro-4 phényl)-1 pipérazinyl-4)-3 propyl]-1 pipérazine dione-2,6

Rendement : 20 %
Point de fusion : 220°C.

EXEMPLE 13 :

Oxalate de la (méthoxy-2 benzyl)-4 [((méthyl-2 phényl)-1 pipérazinyl-4)-3 propyl]-1 pipérazine dione-2,6

Rendement : 47 %
Point de fusion : 203°C.

EXEMPLE 14 :

(méthoxy-2 benzyl)-4 [((méthyl-4 phényl)-1 pipérazinyl-4)-3 propyl]-1 pipérazine dione-2,6

Rendement : 35 %
Point de fusion : 123°C

EXEMPLE 15 :

Dichlorhydrate de la (méthoxy-2 benzyl)-4 [((trifluorométhyl-3 phényl)-1 pipérazinyl-4)-3 propyl]-1 pipérazine dione-2,6

Rendement : 80 %
Point de fusion : 130-140°C

EXEMPLE 16 :

Trichlorhydrate de la (méthoxy-2 benzyl)-4 [((trifluorométhyl-3 phényl)-1 pipérazinyl-4)-2 éthyl]-1 pipérazine dione-2,6

Rendement : 65 %
Point de fusion : 158°C

EXEMPLE 17 :

Trichlorhydrate de la (pyridyl-2 méthyl)-4 [((chloro-2 phényl)-1 pipérazinyl-4)-3 propyl]-1 pipérazine dione-2,6

Rendement : 56 %
Point de fusion : 160°C

EXEMPLE 18 :

Tetrachlorhydrate de la (pyridyl-2 méthyl)-4 [((méthoxy-2 phényl)-1 pipérazinyl-4)-3 propyl]-1 pipérazine dione-2,6

Rendement : 42 %
Point de fusion : 185°C

EXEMPLE 19 :

Trichlorhydrate de la (pyridyl-2 méthyl)-4 [((méthyl-2 phényl)-1 pipérazinyl-4)-3 propyl]-1 pipérazine dione-2,6

Rendement : 48 %
Point de fusion : 190°C

EXEMPLE 20 :

Trichlorhydrate de la (pyridyl-2 méthyl)-4 [((méthyl-4 phényl)-1 pipérazinyl-4)-3 propyl]-1 pipérazine dione-2,6

Rendement : 51 %
Point de fusion : 203°C

EXEMPLE 21 :

Dioxalate de la (pyridyl-2 méthyl)-4 [((trifluorométhyl-3 phényl)-1 pipérazinyl-4)-3 propyl]-1 pipérazine dione-2,6

Rendement : 55 %
Point de fusion : 152°C

EXEMPLE 22 :

Trichlorhydrate de la (pyridyl-3 méthyl)-4 [((chloro-3 phényl)-1 pipérazinyl-4)-3 propyl]-1 pipérazine dione-2,6

Rendement : 45 %
Point de fusion : 130°C

EXEMPLE 23 :

Triméthanesulfonate de la diphénylméthyl-4 [((méthyl-2 phényl)-1 pipérazinyl-4)-3 propyl]-1 pipérazine dione-2,6

Rendement : 54 %
Point de fusion : 80°C

EXEMPLE 24 :

Dimandélate de la diphénylméthyl-4 [((trifluorométhyl-3 phényl)-1 pipérazinyl-4)-3 propyl]-1 pipérazine dione-2,6

Rendement : 48 %
Point de fusion : 60°C

EXEMPLE 25 :

Dimandélate de la (chloro-4 diphénylméthyl)-4 [((fluoro-4 phényl)-1 pipérazinyl-4)-3 propyl]-1 pipérazine dione-2,6

Rendement : 80 %
Point de fusion : 105°C

EXEMPLE 26 :

Dicitrate de la (chloro-4 diphénylméthyl)-4 [((trifluorométhyl-3 phényl)-1 pipérazinyl-4)-3 propyl]-1 pipérazine dione-2,6

Rendement : 70 %
Point de fusion : 80°C

EXEMPLE 27 :

Dichlorhydrate de la cyclohexyl-4 [((trifluorométhyl-3 phényl)-1 pipérazinyl-4)-3 propyl]-1 pipérazine dione-2,6

Rendement : 47 %
Point de fusion : 238°C

EXEMPLE 28 :

Diphosphate de la benzyl-1 [((trifluorométhyl-3 phényl)-1 pipérazinyl-4)-3 propyl]-4 pipérazine dione-2,6

Dans une solution de 0,0166 mole de benzyl-1 pippérazine dione-2,6 et 0,019 mole de (trifluorométhyl-3 phényl)-4 (chloro-3 propyl)-1 pipérazine dans 100 ml de butanone-2, ajouter un mélange de 6 g de carbonate de sodium et 0,4 g d'iodure de sodium. Porter au reflux jusqu'à complète disparition de la pipérazine dione de départ. Refroidir le milieu à la température ambiante, filtrer les sels minéraux et évaporer le solvant sous vide. Reprendre le résidu dans du dichlorométhane, laver avec une solution aqueuse de carbonate de sodium, puis à l'eau et sécher la phase organique sur sulfate de sodium anhydre. Evaporer le solvant sous vide et purifier le résidu par chromatographie sur colonne de silice fine (200 g) en éluant avec un mélange d'éther éthylique et d'acétone (95/5). Evaporer l'éluant et dissoudre les 5 g du produit pur ainsi obtenus dans 21 ml d'une solution d'acide phosphorique M dans l'acétone.
Isoler le diphosphate formé par filtration et le recristalliser dans 140 ml d'éthanol pour obtenir 3,5 g de produit cristallisé.
Rendement : 31 %
Point de fusion : 186°C.

Les constantes physiques spectrales du diphosphate de la benzyl-1 [((trifluorométhyl-3 phényl)-1 pipérazinyl-4)-3 propyl]-4 pipérazine dione-2,6 sont indiquées dans le tableau I.

EXEMPLE 29 :

Diphosphate de la (méthoxy-3 benzyl)-1 [((trifluorométhyl-3 phényl)-1 pipérazinyl-4)-3 propyl]-4 pipérazine dione-2,6

Ce composé a été préparé selon la méthode décrite dans l'exemple 28.
Rendement : 31 %
Point de fusion : 190°C.

Ses constantes physiques spectrales sont indiquées dans le tableau I.

COMPOSES DE FORMULE GENERALE I

| EX. | $R_1$ | A | B | n | $R_2$ | IR $cm^{-1}$ ν (C=O) | RMN (solvant) (b)=base (a)=acide |
|---|---|---|---|---|---|---|---|
| 1 | 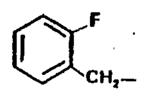 | $-CH_2-$ | >C = O | 3 | 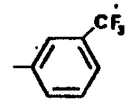 | 1680<br>1735 | ($CDCl_3$) (b)<br>1,5 à 2 ppm,m,2H; 2,3 à 2,8 ppm,m, 6H; 3 à 3,5 ppm,m,4H; 3,45 ppm,s, 4H; 3,75 ppm,s,2H; 3,85 ppm,t,2H; 6,8 à 7,6 ppm,m,8H |
| 2 | 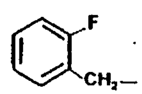 | $-CH_2-$ | >C = O | 3 | 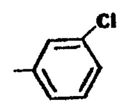 | 1680<br>1735 | ($CDCl_3$) (b)<br>1,5 à 2 ppm,m,2H; 2,4 à 2,8 ppm,m, 6H; 3 à 3,4 ppm,m,,4H; 3,4 ppm,s, 4H; 3,7 ppm,s,2H; 3,8 ppm,t,2H; 6,6 à 7,6 ppm,m,8H |
| 3 | 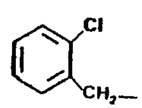 | $-CH_2-$ | >C = O | 3 | 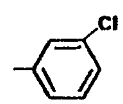 | 1685<br>1735 | ($CDCl_3$) (b)<br>1,5 à 2 ppm,m,2H; 2,2 à 2,8 ppm,m, 6H; 3 à 3,4 ppm,m,4H; 3,45 ppm,s, 4H; 3,7,ppm,s,2H; 3,8 à 4 ppm,m,2H; 6,5 à 7,5 ppm,m,8H |

**COMPOSES DE FORMULE GENERALE I (SUITE 1)**

| EX. | $R_1$ | A | B | n | $R_2$ | IR $cm^{-1}$ ν (C=O) | RMN (solvant) (b)=base (a)=acide |
|---|---|---|---|---|---|---|---|
| 4 | 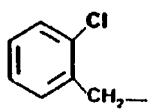 | $-CH_2-$ | $\rangle C=O$ | 3 | 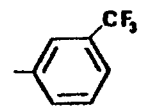 | 1680<br>1735 | ($CDCl_3$) (b)<br>1,5 à 2 ppm,m,2H; 2,2 à 2,7 ppm,m, 6H; 3 à 3,4 ppm,m,4H; 3,4 ppm,s,4H; 3,7 ppm,s,2H; 3,8 ppm,t,2H; 6,8 à 7,6 ppm,m,8H |
| 5 | 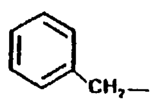 | $-CH_2-$ | $\rangle C=O$ | 4 | 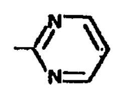 | 1690<br>1750 | ($D_2O$) (a)<br>1,3 à 1,9 ppm,m,4H; 3,1 à 4 ppm,m, 10H; 4,1 ppm,s,4H; 4,3 ppm,s,2H; 4,75 ppm,s,2H; 7,1 ppm,t,1H; 7,6 ppm,s,5H; 8,7 ppm,d,2H; |
| 6 | 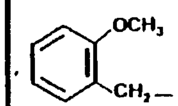 | $-CH_2-$ | $\rangle C=O$ | 4 | 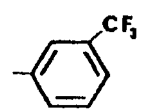 | 1675<br>1730 | ($CDCl_3$) (b)<br>1,2 à 2 ppm,m,4H; 2,2 à 2,9 ppm,m, 6H; 3,1 à 3,5 ppm,m,6H; 3,5 ppm,s, 4H; 3,8 ppm,s,2H; 3,9 ppm,s,3H; 6,8 à 7,7 ppm,m,8H |

COMPOSES DE FORMULE GENERALE I (SUITE 2)

| EX. | $R_1$ | A | B | n | $R_2$ | IR $cm^{-1}$ ν (C=O) | RMN (solvant) (b)=base (a)=acide |
|---|---|---|---|---|---|---|---|
| 7 | 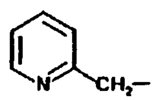 | $-CH_2-$ | $>C=O$ | 3 | 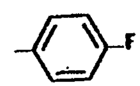 | 1680<br>1730 | ($D_2O$) (a)<br>1,5 à 2,5 ppm,m,2H; 3 à 4,2 ppm,m, 16H; 4,3 ppm,s,2H; 7,2 à 7,4 ppm m,4H; 7,9 à 9 ppm,m,4H |
| 8 | 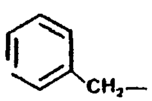 | $-CH_2-$ | $>C=O$ | 3 | 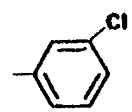 | 1680<br>1730 | (DMSO-$d_6$) (a)<br>1,5 à 2,3 ppm,m,2H; 2,8 à 4,2 ppm, m,18H; 6,7 à 7,6 ppm,m,9H;<br>1H échangeable |
| 9 | 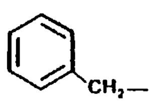 | $-CH_2-$ | $>C=O$ | 3 | 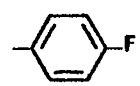 | 1690<br>1740 | (DMSO-$d_6$) (a)<br>1,6 à 2,2 ppm,m,2H; 2,7 à 4, ppm,m, 18H; 6,9 à 7,5 ppm,m,9H;<br>2H échangeables |

COMPOSES DE FORMULE GENERALE I (SUITE 3)

| EX. | $R_1$ | A | B | n | $R_2$ | IR $cm^{-1}$ ν (C=O) | RMN (solvant) (b)=base (a)=acide |
|---|---|---|---|---|---|---|---|
| 10 | 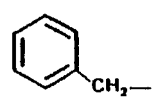 | $-CH_2-$ | $>C = O$ | 2 | 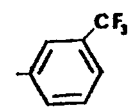 | 1670<br>1730 | ($CDCl_3$) (b)<br>2,4 à 2,9 ppm,m,6H; 3 à 3,4 ppm,m,4H; 3,4 ppm,s,4H; 3,65 ppm,s,2H; 3,95 ppm,t,2H; 6,9 à 7,5 ppm,m,9H |
| 11 | 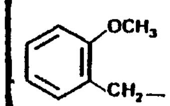 | $-CH_2-$ | $>C = O$ | 3 | 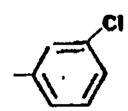 | 1690<br>1745 | ($D_2O$) (a)<br>1,7 à 2,3 ppm,m,2H; 3 à 4 ppm,m,12H; 3,9 ppm,s,3H; 4,35 ppm,s,4H; 4,55 ppm,s,2H; 6,8 à 7,7 ppm,m,8H |
| 12 | 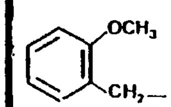 | $-CH_2-$ | $>C = O$ | 3 | 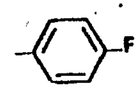 | 1690<br>1750 | (DMSO-$d_6$) (a)<br>1,7 à 2,4 ppm,m,2H; 2,9 à 4 ppm,m,12H; 3,9 ppm,s,3H; 4,2 ppm,s,4H; 4,5 ppm,s,2H; 6,9 à 7,8 ppm,m,8H; 2H échangeables |

## COMPOSES DE FORMULE GENERALE I (SUITE 4)

| EX. | $R_1$ | A | B | n | $R_2$ | IR $cm^{-1}$ ν (C=O) | RMN (solvant) (b)=base (a)=acide |
|---|---|---|---|---|---|---|---|
| 13 | 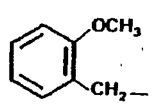 | $-CH_2-$ | $>C=O$ | 3 | 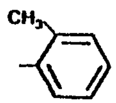 | 1600<br>1740 | ($DMSO-d_6$) (a)<br>1,6 à 2,2 ppm,m,2H; 2,3 ppm,s,3H; 2,7 à 3,8 ppm,m,18H; 3,8 ppm,s,3H; 6,8 à 7,5 ppm,m,8H; 2H échangeables |
| 14 | 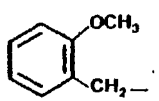 | $-CH_2-$ | $>C=O$ | 3 | 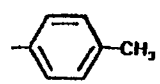 | 1680<br>1730 | ($CDCl_3$) (b)<br>1,5 à 2 ppm,m,2H; 2,25 ppm,s,3H; 2,3 à 2,7 ppm,m,6H; 2,9 à 3,2 ppm, m,6H; 3,7 ppm,s,2H; 3,4 ppm,s,4H; 3,8 ppm,s,3H; 6,7 à 7,4 ppm,m,8H |
| 15 | 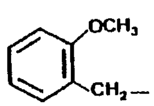 | $-CH_2-$ | $>C=O$ | 3 | 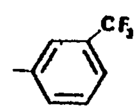 | 1680<br>1735 | ($CDCl_3$) (b)<br>1,5 à 2 ppm,m,2H; 2,2 à 2,9 ppm,m, 6H; 3 à 4 ppm,m,15H; 6,7 à 7,6 ppm, m,8H |

## COMPOSES DE FORMULE GENERALE I (SUITE 5)

| EX. | $R_1$ | A | B | n | $R_2$ | IR $cm^{-1}$ ν (C=O) | RMN (solvant) (b)=base (a)=acide |
|---|---|---|---|---|---|---|---|
| 16 | 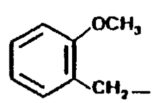 | $-CH_2-$ | $>C = O$ | 2 | 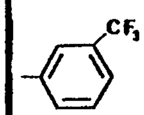 | 1690<br>1740 | $(DMSO + CDCl_3)$ (a)<br>3 à 4,5 ppm,m,12H; 3,9 ppm,s,4H;<br>4,5 ppm,s,3H; 4,55 ppm,s,2H;<br>6,7 à 7,9 ppm,m,8H; 3H échangeables |
| 17 | 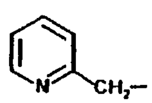 | $-CH_2-$ | $>C = O$ | 3 | 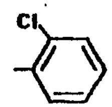 | 1680<br>1730 | $(D_20)$ (a)<br>1,6 à 2,2 ppm,m,2H; 2,9 à 4,1 ppm,<br>m,16H; 4,2 ppm,s,2H; 7 à 7,6 ppm,m,<br>4H; 7,8 à 8,9 ppm,m,4H |
| 18 | 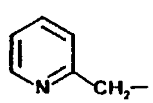 | $-CH_2-$ | $>C = O$ | 3 | 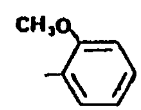 | 1680<br>1730 | $(D_2O)$ (a)<br>1,8 à 2,6 ppm,m,2H; 3,3 à 4,3 ppm<br>m,16H; 3,9 ppm,s,3H; 4,3 ppm,s,2H;<br>7 à 8 ppm,m,4H; 8 à 9,1 ppm,m,4H |

COMPOSES DE FORMULE GENERALE I (SUITE 6)

| EX. | $R_1$ | A | B | n | $R_2$ | IR $cm^{-1}$ ν (C=O) | RMN (solvant) (b)=base (a)=acide |
|---|---|---|---|---|---|---|---|
| 19 | 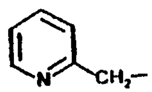 | $-CH_2-$ | $>C=O$ | 3 | 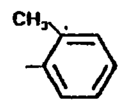 | 1680<br>1730 | (DMSO-$d_6$) (a)<br>2,3 ppm,s,5H; 2,8 à 4 ppm,m,12H; 3,8 ppm,s,4H; 4,3 ppm,s,2H; 6,9 à 7,3 ppm,m,4H; 7,7 à 9 ppm,m,4H; 3H échangeables |
| 20 | 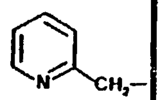 | $-CH_2-$ | $>C=O$ | 3 | 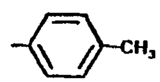 | 1680<br>1730 | (DMSO-$d_6$) (a)<br>1,8 à 3 ppm,m,5H; 3 à 4 ppm,m,12H; 3,8 ppm,s,4H; 4,35 ppm,s,2H; 6,9 à 7,3 ppm,m,4H; 7,5 à 9 ppm,m,4H; 3H échangeables |
| 21 | 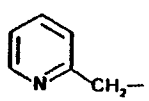 | $-CH_2-$ | $>C=O$ | 3 | 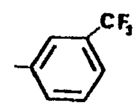 | 1680<br>1730 | (DMSO-$d_6$) (a)<br>1,5 à 2,1 ppm,m,2H; 2,6 à 3,9 ppm, m,12H; 3,8 ppm,s,4H; 4,3 ppm,s,2H; 6,9 à 7,9 ppm,m,7H; 8,5 ppm,d,1H; 4H échangeables |

## COMPOSES DE FORMULE GENERALE I (SUITE 7)

| EX. | $R_1$ | A | B | n | $R_2$ | IR $cm^{-1}$ ν (C=O) | RMN (solvant) (b)=base (a)=acide |
|---|---|---|---|---|---|---|---|
| 22 | 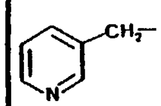 | $-CH_2-$ | >C = O | 3 | 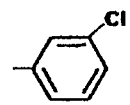 | 1695<br>1750 | ($DMSO-d_6$) (a)<br>1,6 à 2,3 ppm,m,2H; 2,7 à 4 ppm,m, 10H; 4 ppm,s,4H; 4 à 4,5 ppm,m,2H; 4,35 ppm,s,2H; 6,6 à 9,2 ppm,m,8H; 3H échangeables |
| 23 | 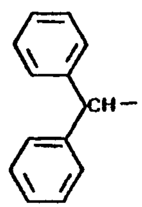 | $-CH_2-$ | >C = O | 3 | 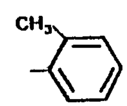 | 1680<br>1740 | ($CDCl_3$) (b)<br>1,6 à 1,95 ppm,m,2H; 2,25 ppm,s,3H; 2,3 à 3,1 ppm,m,10H; 3,3 ppm,s,4H; 3,8 ppm,t,2H; 4,3 ppm,s,1H; 6,9 à à 7,6 ppm,m,14H |
| 24 | 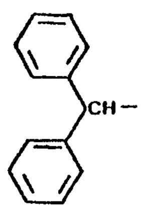 | $-CH_2-$ | >C = O | 3 | 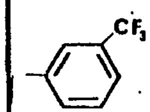 | 1680<br>1735 | ($CDCl_3$) (a)<br>1,5 à 2,3 ppm,m,2H; 2,6 à 3,2 ppm, m,10H; 3,3 ppm,s,4H; 3,65 ppm,t, 2H; 4,3 ppm,s,1H; 5 ppm,s,2H; 6,7 à 7,7 ppm,m,24H; 4H échangeables |

COMPOSES DE FORMULE GENERALE I (SUITE 8)

| EX. | $R_1$ | A | B | n | $R_2$ | IR $cm^{-1}$ ν (C=O) | RMN (solvant) (b)=base (a)=acide |
|---|---|---|---|---|---|---|---|
| 25 | 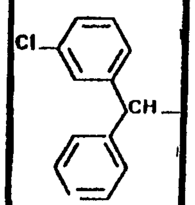 | $-CH_2-$ | $>C=O$ | 3 | 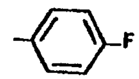 | 1675 1735 | ($CDCl_3$) (b) 1,5 à 1,7 ppm,m,2H; 2,2 à 2,5 ppm, m,6H; 2,9 à 3,1 ppm,m,4H; 3,2 ppm, s,4H; 3,7 ppm,t,2H; 4,2 ppm,s,1H; 6,8 à 7,2 ppm,m,13H |
| 26 | 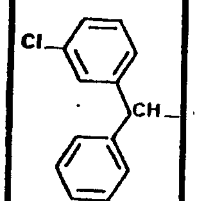 | $-CH_2-$ | $>C=O$ | 3 | 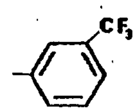 | 1680 1735 | ($CDCl_3$) (b) 1,5 à 2,3 ppm,m,2H; 2,3 à 2,7 ppm, m,6H; 3 à 3,5 ppm,m,4H; 3,8 ppm,s, 4H; 4 ppm,t,2H; 4,4 ppm,s,1H; 7 à 7,7 ppm,m,13H |
| 27 | 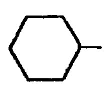 | $-CH_2-$ | $>C=O$ | 3 | 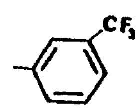 | 1690 1740 | (DMSO-$d_6$) (a) 1 à 2,4 ppm,m,12H; 3 à 4,2 ppm,m, 13H; 4,35 ppm,s,4H; 7 à 7,8 ppm,m, 4H; 2H échangeables |

**COMPOSES DE FORMULE GENERALE I (SUITE 9)**

| EX. | $R_1$ | A | B | n | $R_2$ | IR $cm^{-1}$ (C=O) | RMN (solvant) (b)=base (a)=acide |
|---|---|---|---|---|---|---|---|
| 28 | 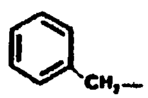 | ⟩ C = O | $-CH_2-$ | 3 | 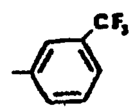 | 1700<br>1740 | ($DMSO-d_6$) (a)<br>1,5 à 2,1 ppm,m,2H; 2,5 à 3,5 ppm, m,16H; 4,8 ppm,s,2H; 7 à 7,5 ppm, m,9H; 6H échangeables |
| 29 | 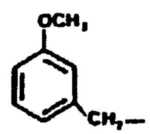 | ⟩ C = O | $-CH_2-$ | 3 | 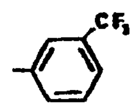 | 1695<br>1740 | (DMSO $d_6$ + $CDCl_3$) (a)<br>1,7 à 2,3 ppm,m,2H; 2,3 à 4 ppm, m,16H; 3,7 ppm,s,3H; 4,8 ppm,s, 2H; 6,6 à 7,6,m,8H; 6H échangeables |

ETUDE PHARMACOLOGIQUE

EXEMPLE 30 :

Evaluation de l'activité anxiolytique par le test des quatre plaques

L'activité anxiolytique des composés de la formule générale I a été recherchée chez la souris selon la méthode de Aron Simon Larousse et Boissier décrite dans Neuropharmacology (1971) 10 p. 459-469. Après traitement des animaux au moyen de différentes doses des composés de l'invention administrées par voie intrapéritonéale ou orale, l'augmentation du pourcentage des transitions lors de l'exploration du fond de la cage-test, en présence de chocs électriques, a été évaluée. Les résultats de cette étude sont indiqués dans les tableaux II et III.

TABLEAU II

| EXEMPLE | DOSE i.p. (mg.kg$^{-1}$) | % D'AUGMENTATION DES REPONSES (TRANSITIONS) |
|---|---|---|
| 1 | 5 | 65 |
| 4 | 5 | 37 |
| | 25 | 67 |
| 6 | 25 | 37 |
| 10 | 25 | 85 |
| 15 | 20 | 52 |
| 16 | 10 | 41 |
| 21 | 5 | 69 |
| 22 | 10 | 44 |
| 23 | 10 | 47 |
| | 25 | 77 |
| 24 | 25 | 62 |
| 27 | 25 | 73 |
| 28 | 25 | 82 |
| 29 | 25 | 49 |

TABLEAU III

| EXEMPLE | DOSE p.o. ($mg.kg^{-1}$) | % D'AUGMENTATION DES REPONSES |
|---|---|---|
| 1 | 50 | 64 |
| 4 | 25 | 35 |
| | 50 | 58 |
| 15 | 10 | 61 |
| | 50 | 90 |
| 26 | 25 | 69 |

EXEMPLE 31 :

Evaluation de l'activité anxiolytique par le test de "l'escalier"

L'activité anxiolytique des composés de l'invention a été recherchée chez le rat selon la méthode décrite par Thiebot, Soubrié et Boissier dans J. Pharmacol. 1976, 7,(1), p. 87-102.

Dans ce test, le comportement des redressements est associé à un état d'angoisse de l'animal. Le nombre de marches gravies représente le comportement exploratoire normal du rat introduit dans l'enceinte. Normalement, un composé anxiolytique efficace doit diminuer, pendant le test qui dure 3 minutes, le nombre des redressements sans modifier le nombre de marches gravies, ou au contraire augmenter ce dernier paramètre.

Comme les résultats indiqués dans le tableau IV le démontrent, les composés de formule générale I diminuent significativement le nombre de redressements sans pour autant modifier le nombre de marches gravies.

TABLEAU IV

| EXEMPLE | DOSE i.p. ($mg.kg^{-1}$) | % DE VARIATION DES REDRESSEMENTS | % DE VARIATION DES MARCHES GRAVIES |
|---|---|---|---|
| 1 | 2,5 | - 20 | NS |
| | 10 | - 39 | NS |
| | 20 | - 64 | NS |
| 4 | 10 | - 47 | NS |
| 5 | 1,25 | - 40 | NS |
| 15 | 10 | - 43 | NS |
| | 20 | - 64 | NS |
| 20 | 2,5 | - 43 | NS |
| 27 | 2,5 | - 41 | NS |

NS : non significatif

EXEMPLE 32 :

Evaluation de l'activité anxiolytique pa le test de Conflit de Mac Millan

Dans ces expériences de conditionnement opérant en présence d'une récompense alimentaire, effectuées chez le rat, selon la méthode décrite par Mac Millan dans Fed. Proceedings, 1975, 34,(9), p. 1870-1879, on considère deux paramètres dans les réponses comportementales de l'animal. Le premier paramètre est la variation du taux de réponses punies, en présence d'un choc électrique, sous l'influence du traitement par rapport au taux de réponses punies sous l'influence du sérum physiologique. Le deuxième paramètre est la variation du taux de réponses non punies, sans choc électrique, sous l'influence du même traitement, par rapport au taux de réponses non punies, lors de l'administration de sérum physiologique. Un effet anxiolytique spécifique est constaté lorsqu'après l'administration d'une substance le taux des seules réponses punies se trouve augmenté. Les composés de formule générale I donnent des résultats positifs sur ce test comme il est indiqué sur le tableau V.

TABLEAU V

| EXEMPLE | DOSE i.p. ($mg.kg^{-1}$) | % DE VARIATION DES REPONSES PUNIES | % DE VARIATION DES REPONSES NON PUNIES |
|---|---|---|---|
| 1 | 2,5 | + 49 S | + 18 NS |
| 10 | 5 | + 67 S | + 18 NS |
| 14 | 5 | + 48 S | + 14 NS |
| 15 | 10 | + 41 S | + 37 NS |

S = significatif
NS = non significatif

EXEMPLE 33 :

Evaluation de l'activité anxiolytique par le test de Geller-Seifter

L'activité anxiolytique des composés de la présente invention a été évaluée aussi chez le rat avec le test de Geller-Seifter décrit par Geller, Kulac et Seifter dans Psychopharmacologia, 1962, 3, p. 374-375.

Comme dans le cas du test précédent, les paramètres de réponses punies, (avec choc-électrique), et non punies, (sans choc), sont pris en considération pour la mise en évidence d'un effet anxiolytique des substances étudiées. Une augmentation portant uniquement sur les taux des réponses punies détermine l'existence d'un effet anxiolytique authentique.

Les composés de la présente invention affectent de façon significative le taux de réponses punies. A titre d'exemple, le composé de l'exemple 1 à la dose de 2,5 $mg.kg^{-1}$ (ip) et le composé de l'exemple 2 à la dose de 5,0 $mg.kg^{-1}$ (ip) augmentent les réponses punies de 39 % ($p < 0,05$) et de 65 % ($p < 0,05$) respectivement.

EXEMPLE 34

Inhibition de l'agressivité d'isolement chez la souris

Les composés de formule générale I inhibent le comportement d'attaque des souris rendues agressives par isolement selon la méthode décrite par Yen, Stanger et Millman dans Arch. Int. Pharmacodyn. 1959, 123, p. 179-185. La dose efficace (i.p.) inhibant 50 % des animaux agressifs ($DE_{50}$) est indiquée dans le tableau VI.

TABLEAU VI

| EXEMPLE | $DE_{50}$ ($mg.kg^{-1}$) voie IP inhibitrice |
|---|---|
| 1 | 7,64 |
| 6 | 4,74 |
| 9 | 8,60 |
| 17 | 6,11 |
| 19 | 6,78 |
| 21 | 11,08 |

EXEMPLE 35 :

Inhibition de l'agressivité du rat isolé et bulbectomisé

Les composés de la présente invention inhibent l'agressivité du rat isolé et bulbectomisé. L'inhibition de l'agressivité a été évaluée selon la méthode décrite par Vergnes et Karli dans C.R.Soc.Biol. 1963, 157, p. 1061. L'effet sur l'agressivité est évalué par le pourcentage des animaux devenus non tueurs après traitement. Les résultats de cet essai sont indiqués dans le tableau VII.

TABLEAU VII

| EXEMPLE | DOSE i.p. ($mg.kg^{-1}$) | % DES ANIMAUX NON TUEURS |
|---|---|---|
| 1 | 25 | 45 |
| 7 | 25 | 67 |
| 9 | 25 | 60 |
| 10 | 25 | 66 |
| 15 | 25 | 64 |
| 21 | 12,5 | 73 |
| 22 | 12,5 | 83 |

EXEMPLE 36 :

Inhibition de la réponse conditionnée d'évitement actif chez le rat

Les résultats obtenus avec les composés de formule générale I au cours des différentes expérimentations mettant en oeuvre la méthode décrite par Courvoisier et coll. dans Arch. Int. Pharmacodyn., 1953, 92, p. 305-361 et par Janssen et coll. dans Arzneim. Forsch,1965, 15, p. 104-117 sont rapportés dans le tableau X. Les pourcentages d'inhibition de la réponse conditionnée d'évitement obtenus (% IRCE) démontrent que les composés de l'invention possèdent une propriété antipsychotique éventuelle intéressante.

**TABLEAU X**

| EXEMPLE | DOSE i.p. ($mg.kg^{-1}$) | % I.R.C.E. |
|---|---|---|
| 7 | 10 | 30 |
| | 15 | 48 |
| | 20 | 79 |
| 18 | 10 | 18 |
| | 15 | 35 |
| | 20 | 56 |
| 19 | 20 | 26 |
| | 40 | 73 |

EXEMPLE 37 :

Recherche des effets secondaires

Le test de la "tige tournante" (actionnée à la vitesse de 4 tours/minute) effectué chez la souris a été utilisé pour rechercher l'effet myorelaxant. Les composés de l'invention administrés 30 minutes avant cet essai, à la dose de 25 $mg.kg^{-1}$ et de 50 $mg.kg^{-1}$ par voie intrapéritonéale, n'augmentent pas d'une manière significative le pourcentage des chutes des animaux traités par rapport à celles observées chez les témoins non traités.

L'effet anti-convulsivant des composés de l'invention a été recherché chez la souris par le test de l'électrochoc ainsi qu'en recherchant un antagonisme vis à vis des convulsions induites par le pentylènetetrazol.

Les composés de formule générale I administrés à la dose de 25 $mg.kg^{-1}$ par voie intrapéritonéale ne protègent pas les animaux des convulsions induites par l'électrochoc. De même, les différents composés de l'invention à la dose de 50 $mg.kg^{-1}$ n'antagonisent les convulsions induites par une dose de 100 $mg.kg^{-1}$ de pentylènetétrazol administrée par voie intrapéritonéale.

PREPARATION PHARMACEUTIQUE

EXEMPLE 38

Gélules dosées à 2 mg de dimandélate de la (fluoro-2 benzyl)-4 [((trifluorométhyl-3 phényl)-1 pipérazinyl-4)-3 propyl]-1 pipérazine dione-2,6

(fluoro-2 benzyl)-4 [((trifluorométhyl-3 phényl)-1 pipérazinyl-4)-3 propyl]-1 pipérazine dione-2,6  2mg
Amidon de maïs  15 mg
Lactose  25 mg
Talc  5 mg

**Revendications pour les Etats contractants BE, CH, DE, FR, GB, IT, LI, LU, NL, SE**

1. Composés de formule générale I

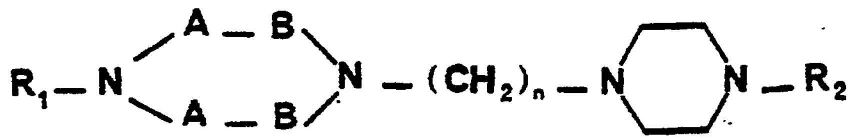

(I)

dans laquelle:
- A et B représentent chacun un radical méthylène ou un radical carbonyle, à condition toutefois que A et B ne représentent jamais simultanément le même radical,
- $R_1$ représente un radical diphénylméthyle éventuellement substitué sur les cycles benzèniques par un atome d'halogène, un radical cyclohexyle, un groupe pyridylméthyle, ou un radical benzyle éventuellement substitué par un atome d'halogène ou par un radical alcoxy renfermant de 1 à 4 atomes de carbone,
- $R_2$ représente un radical pyrimidinyle ou un radical phényle éventuellement substitué par un atome d'halogène, par un radical alkyle de 1 à 4 atomes de carbone, par un radical trifluorométhyle ou par un radical alcoxy renfermant de 1 à 4 atomes de carbone,
- n est un nombre entier pouvant prendre les valeurs de 2 à 4,

et leurs sels formés par addition à un acide minéral ou organique pharmaceutiquement acceptable.

2. La (fluoro-2 benzyl)-4 [((trifluorométhyl-3 phényl)-1 pipérazinyl-4)-3 propyl]-1 pipérazine dione-2,6 et ses sels d'addition avec un acide minéral ou organique pharmaceutiquement acceptable.

3. La (méthoxy-2 benzyl)-4 [((trifluorométhyl-3 phényl)-1 pipérazinyl-4)-3 propyl]-1 pipérazine dione-2,6 et ses sels d'addition avec un acide minéral ou organique pharmaceutiquement acceptable.

4. La (pyridyl-2 méthyl)-4 [((trifluorométhyl-3 phényl)-1 pipérazinyl-4)-3 propyl]-1 pipérazine dione-2,6 et ses sels d'addition avec un acide minéral ou organique pharmaceutiquement acceptable.

5. La benzyl-4 [((fluoro-4 phényl)-1 pipérazinyl-4)-3 propyl]-1 pipérazine dione-2,6 et ses sels d'addition avec un acide minéral ou organique pharmaceutiquement acceptable.

6. La (méthoxy-2 benzyl)-4 [((fluoro-4 phényl)-1 pipérazinyl-4)-3 propyl]-1 pipérazine dione-2,6 et ses sels d'addition avec un acide minéral ou organique pharmaceutiquement acceptable.

7. La (méthoxy-2 benzyl)-4 [((trifluorométhyl-3 phényl)-1 pipérazinyl-4)-4 butyl]-1 pipérazine dione-2,6 et ses sels d'addition avec un acide minéral ou organique pharmaceutiquement acceptable.

8. La (pyridyl-2 méthyl)-4 [((chloro-2 phényl)-1 pipérazinyl-4)-3 propyl]-1 pipérazine dione-2,6 et ses sels d'addition avec un acide minéral ou organique pharmaceutiquement acceptable.

9. La (pyridyl-2 méthyl)-4 [((méthyl-2 phényl)-1 pipérazinyl-4)-3 propyl]-1 pipérazine dione-2,6 et ses sels d'addition avec un acide minéral ou organique pharmaceutiquement acceptable.

10. La (chloro-2 benzyl)-4 [((trifluorométhyl-3 phényl)-1 pipérazinyl-4)-3 propyl]-1 pipérazine dione-2,6 et ses sels d'addition avec un acide minéral ou organique pharmaceutiquement acceptable.

11. La (méthoxy-3 benzyl)-1 [((trifluorométhyl-3 phényl)-1 pipérazinyl-4)-3 propyl]-4 pipérazine dione-2,6 et ses sels d'addition avec un acide minéral ou organique pharmaceutiquement acceptable.

12. Procédé de préparation des composés de formule générale I, caractérisé en ce que :
- soit :
l'on condense une imide de formule générale II :

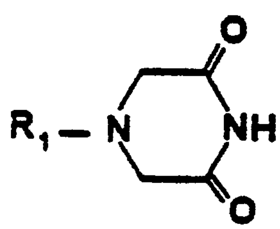

(II)

dans laquelle la définition du substituant $R_1$ demeure celle mentionnée précédemment, avec un composé de formule générale III :

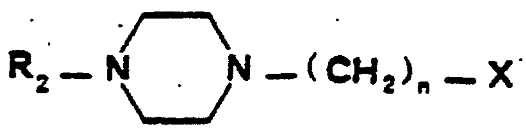

(III)

dans laquelle $R_2$ est tel que précédemment défini, n représente 2 ou 3 et X représente un atome de chlore ou de brome, dans un solvant organique anhydre polaire à une température comprise entre 40° et 100°C et en présence d'un hydrure métallique, tel que l'hydrure de sodium et d'un sel minéral tel que l'iodure de sodium pour obtenir les composés de formule I dans laquelle la définition de $R_1$ et $R_2$ demeure celle indiquée précédemment, n est égal à 2 ou 3, A représente un méthylène et B un carbonyle,
ou
l'on fait réagir sur le composé de formule générale II le bromo-1 chloro-3 propane dans un solvant organique anhydre polaire tel que la butanone-2 en présence d'un hydrure métallique tel que l'hydrure de sodium pour obtenir un composé de formule générale IV :

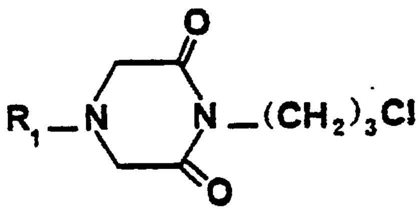

(IV)

dans laquelle $R_1$ a la signification précédemment définie pour la formule I,
et ensuite l'on condense la (chloro-3 propyl)-1 pipérazine dione-2,6 ainsi obtenue avec une pipérazine de formule générale V :

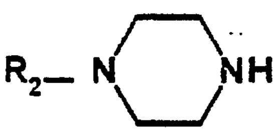

(V)

dans laquelle la définition de $R_2$ demeure celle indiquée précédemment dans un solvant organique polaire tel que la butanone-2 en présence de sels minéraux tels que le carbonate de sodium et l'iodure de sodium et à une température comprise entre 40°C et 100°C, pour obtenir un composé de formule générale I dans laquelle $R_1$ et $R_2$ ont les significations précédemment définies, n est égal à 3, A représente un méthylène et B un carbonyle,
- <u>soit</u> :
l'on condense une morpholine dione-2,6 de formule générale VI :

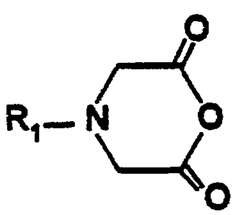

(VI)

dans laquelle $R_1$ a la signification précédemment définie pour la formule I, avec une pipérazinyl-4 butylamine de formule générale VII :

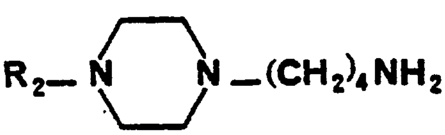

(VII)

dans laquelle la définition de $R_2$ reste identique à celle donnée pour la formule I, dans un solvant organique basique tel que la pyridine et à une température comprise entre 40° et 100°C,
pour obtenir un composé de formule I dans laquelle la définition de $R_1$ et $R_2$ demeure celle indiquée précédemment, n est égal à 4, A représente un méthylène et B un carbonyle,
- soit :
l'on condense une pipérazine dione de formule générale VIII :

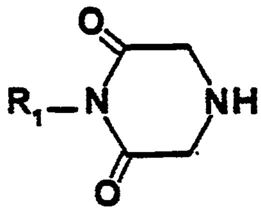

(VIII)

dans laquelle $R_1$ a la signification précédemment définie pour la formule I, avec un dérivé de pipérazine de formule générale III, dans un solvant organique polaire tel que la butanone-2 en présence des sels minéraux tels que le carbonate de sodium et l'iodure de sodium à une température comprise entre 40° et 100°C, pour obtenir un composé de formule générale I dans laquelle $R_1$, $R_2$ et n ont les significations précédemment définies, A représente un carbonyle et B représente un méthylène,
et ensuite si on le désire,
que l'on transforme en un sel d'addition avec un acide organique ou minéral pharmaceutiquement acceptable.

13. Composition pharmaceutique contenant comme principe actif un composé selon l'une quelconque des revendications 1 à 11, en association ou en mélange avec un excipient ou un véhicule inerte non toxique, pharmaceutiquement acceptable.

14. Composition pharmaceutique selon la revendication 13 renfermant le principe actif à la dose de 0,1 à 100 mg.

15. Composition pharmaceutique selon les revendications 13 et 14 renfermant comme principe actif au moins un composé selon les revendications 1 à 11 utilisable dans le traitement des maladies nécessitant des médications anxiolytiques, antiagressives, ou antipsychotiques.

**Revendications pour l'Etat contractant: AT**

1. Procédé de préparation des composés de formule générale I

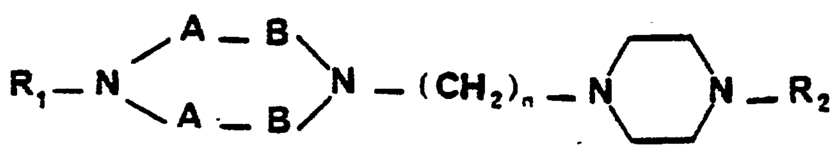

(I)

dans laquelle:
- A et B représentent chacun un radical méthylène ou un radical carbonyle, à condition toutefois que A et B ne représentent jamais simultanément le même radical,
- $R_1$ représente un radical diphénylméthyle éventuellement substitué sur les cycles benzèniques par un atome d'halogène, un radical cyclohexyle, un groupe pyridylméthyle, ou un radical benzyle éventuellement substitué par un atome d'halogène ou par un radical alcoxy renfermant de 1 à 4 atomes de carbone,
- $R_2$ représente un radical pyrimidinyle ou un radical phényle éventuellement substitué par un atome d'halogène, par un radical alkyle de 1 à 4 atomes de carbone, par un radical trifluorométhyle ou par un radical alcoxy renfermant de 1 à 4 atomes de carbone,
- n est un nombre entier pouvant prendre les valeurs de 2 à 4,
et leurs sels formés par addition à un acide minéral ou organique pharmaceutiquement acceptable,
caractérisé en ce que :
- soit :
l'on condense une imide de formule générale II :

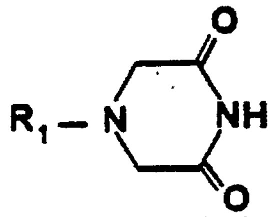

(II)

dans laquelle la définition du substituant $R_1$ demeure celle mentionnée précédemment, avec un composé de formule générale III :

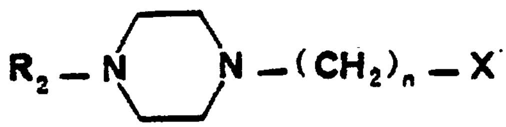

(III)

dans laquelle $R_2$ est tel que précédemment défini, n représente 2 ou 3 et X représente un atome de chlore ou de brome, dans un solvant organique anhydre polaire à une température comprise entre 40° et 100°C et en présence d'un hydrure métallique, tel que l'hydrure de sodium et d'un sel minéral tel que l'iodure de sodium pour obtenir les composés de formule I dans laquelle la définition de $R_1$ et $R_2$ demeure celle indiquée précédemment, n est égal à 2 ou 3, A représente un méthylène et B un carbonyle,
ou
l'on fait réagir sur le composé de formule générale II le bromo-1 chloro-3 propane dans un solvant organique anhydre polaire tel que la butanone-2 en présence d'un hydrure métallique tel que l'hydrure de sodium pour obtenir un composé de formule générale IV :

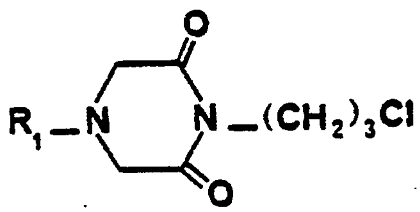

(IV)

dans laquelle $R_1$ a la signification précédemment définie pour la formule I,
et ensuite l'on condense la (chloro-3 propyl)-1 pipérazine dione-2,6 ainsi obtenue avec une pipérazine de formule générale V :

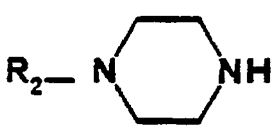

(V)

dans laquelle la définition de $R_2$ demeure celle indiquée précédemment dans un solvant organique polaire tel que la butanone-2 en présence de sels minéraux tels que le carbonate de sodium et l'iodure de sodium et à une température comprise entre 40°C et 100°C, pour obtenir un composé de formule générale I dans laquelle $R_1$ et $R_2$ ont les significations précédemment définies, n est égal à 3, A représente un méthylène et B un carbonyle,
- soit :
l'on condense une morpholine dione-2,6 de formule générale VI :

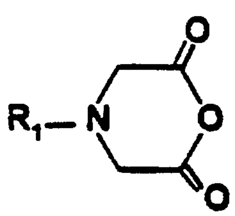

(VI)

dans laquelle $R_1$ a la signification précédemment définie pour la formule I, avec une pipérazinyl-4 butylamine de formule générale VII :

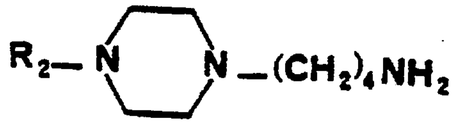

(VII)

dans laquelle la définition de $R_2$ reste identique à celle donnée pour la formule I, dans un solvant organique basique tel que la pyridine et à une température comprise entre 40° et 100°C,
pour obtenir un composé de formule I dans laquelle la définition de $R_1$ et $R_2$ demeure celle indiquée précédemment, n est égal à 4, A représente un méthylène et B un carbonyle,
- soit :
l'on condense une pipérazine dione de formule générale VIII :

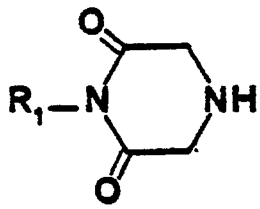

(VIII)

dans laquelle $R_1$ a la signification précédemment définie pour la formule I, avec un dérivé de pipérazine de formule générale III, dans un solvant organique polaire tel que la butanone-2 en présence des sels minéraux tels que le carbonate de sodium et l'iodure de sodium à une température comprise entre 40° et 100°C, pour obtenir un composé de formule générale I dans laquelle $R_1$, $R_2$ et n ont les significations précédemment définies, A représente un carbonyle et B représente un méthylène,
et ensuite si on le désire,
que l'on transforme en un sel d'addition avec un acide organique ou minéral pharmaceutiquement acceptable.

**Patentansprüche für die Vertragsstaaten: BE, CH, DE, FR, IT, LI, LU, NL, SE**

1. Verbindungen der allgemeinen Formel (I)

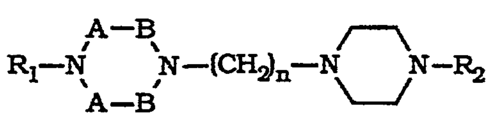

(I)

worin
– A und B jeweils eine Methylengruppe oder eine Carbonylgruppe mit der Maßgabe, daß A und B nicht gleichzeitig die gleiche Gruppe darstellen,
– $R_1$ eine Diphenylmethylgruppe, die gegebenenfalls an den Benzolringen durch ein Halogenatom, eine Cyclohexylgruppe, eine Pyridylmethylgruppe oder eine gegebenenfalls durch ein Halogenatom oder eine Alkoxygruppe mit 1 bis 4 Kohlenstoffatomen substituierte Benzylgruppe substituiert ist,
– $R_2$ eine Pyrimidinylgruppe oder eine Phenylgruppe, die gegebenenfalls durch ein Halogenatom, eine Alkylgruppe mit 1 bis 4 Kohlenstoffatomen, eine Trifluormethylgruppe oder eine Alkoxygruppe mit 1 bis 4 Kohlenstoffatomen substituiert ist, und
– n eine ganze Zahl mit einem Wert von 2 bis 4 bedeuten, und deren durch Addition einer anorganische oder organischen pharmazeutischen annehmbaren Säure gebildete Salze.

2. 4-(2-Fluor-benzyl)-1-[3-(3-trifluormethylphenyl)-4-piperazinyl)-propyl]-piperazin-2,6-dion und dessen Additionssalze mit einer anorganischen oder organischen pharmazeutisch annehmbaren Säure.

3. 4-(2-Methoxy-benzyl)-1-[3-(1-(3-trifluormethyl-phenyl)-4-piperazinyl-propyl]-piperazin-2,6-dion und dessen Aditionssalze mit einer anorganischen oder organischen pharmazeutisch annehmbaren Säure.

4. 4-(2-Pyridyl-methyl)-1-[3-(1-(3-trifluormethyl-phenyl)-4-piperazinyl)-propyl]-piperazin-2,6-dion und dessen Additonssalze mit einer anorganischen oder organischen pharmazeutisch annehmbaren Säure.

5. 4-Benzyl-1-[3-(1-(4-fluor-phenyl)-4-piperazinyl)-propyl]-piperazin-2,6-dion und dessen Additionssalze mit einer anorganischen oder organischen pharmazeutisch annehmbaren Säure.

6. 4-(2-Methoxy-benzyl)-1-[3-(1-(4-fluor-phenyl)-4-piperazinyl)-propyl]-piperazin-2,6-dion und dessen Additionssalze mit einer anorganischen oder organischen pharmazeutisch annehmbaren Säure.

7. 4-(2-Methoxy-benzyl)-1-[3-(1-(3-trifluormethyl-phenyl)-4-piperazinyl)-butyl]-piperazin-2,6-dion und dessen Additionssalze mit einer anorganischen oder organischen pharmazeutisch annehmbaren Säure.

8. 4-(2-Pyridyl-methyl)-1-[3-(1-(2-chlor-phenyl)-4-piperazinyl)-propyl]-piperazin-2,6-dion und dessen Additionssalze mit einer anorganischen oder organischen pharmazeutisch annehmbaren Säure.

9. 4-(2-Pyridyl-methyl)-1-[3-(1-(2-methyl-phenyl)-4-piperazinyl)-propyl]-piperazin-2,6-dion und dessen Addtionssalze mit einer anorganischen oder organischen pharmazeutisch annehmbaren Säure.

10. 4-(2-Chlor-benzyl)-1-[3-(1-(3-trifluormethyl-phenyl)-4-piperazinyl)-propyl]-piperazin-2,6-dion und dessen Additionssalze mit einer anorganischen oder organischen pharmazeutisch annehmbaren Säure.

11. 1-(3-Mthoxy-benzyl)-4-[3-(1-(3-trifluormethyl-phenyl)-4-piperazinyl)-propyl]-piperazin-2,6-dion und dessen Additionssalze mit einer anorganische oder organischen pharmazeutisch annehmbaren Säure.

12. Verfahren zur Herstellung der Verbindung der allgemeinen Formel (I), dadurch gekennzeichnet, daß man
– entweder:
ein Imid der allgemeinen Formel (II)

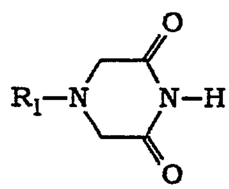

(II)

in der der Substituent $R_1$ die oben angegebenen Bedeutungen besitzt, mit einer Verbindung der allgemeinen Formel (III)

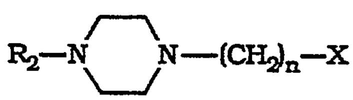

(III)

in der $R_2$ die oben angegebenen Bedeutungen besitzt und n 2 oder 3 und X ein Chloratom oder ein Bromatom bedeuten, in einem wasserfreien, polaren organischen Lösungsmittel bei einer Temperatur zwischen 40 und 100°C und in Gegenwart eines Metallhydrids, wie Natriumhydrid, und eines anorganischen Salzes, wie Natriumjodid, kondensiert zur Bildung der Verbindung der allgemeinen Formel (I), in der $R_1$ und $R_2$ die oben angegebenen Bedeutungen besitzen und n 2 oder 3, A eine Methylengruppe und B eine Carbonylgruppe bedeuten,
– oder die Verbindung der allgmeinen Formel (II) in einem wasserfreien polaren organischen Lösungsmittel, wie Butanon-2, in Gegenwart eines Metallhydrids, wie Natriumhydrid, mit 1-Brom-3-chlor-propan umsetzt zur Bildung einer Verbindung der allgemeinen Formel (IV)

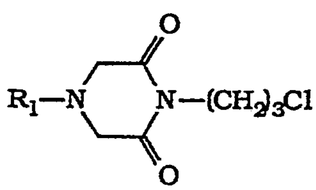

(IV)

in der $R_1$ die oben bezüglich der Formel (I) angegebenen Bedeutungen besitzt, und anschließend das in dieser Weise erhaltene 1-(3-chlor-propyl)-piperazin-2,6-dion mit einem Piperazin der allgemeinen Formel (V)

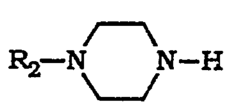

(V)

in der $R_2$ die oben angegebenen Bedeutungen besitzt, in einem polaren organischen Lösungsmittel, wie Butanon-2, in Gegenwart von anorganischen Salzen, wie Natriumcarbonat und Natriumjodid, bei einer Temperatur zwischen 40 und 100°C kondensiert zur Bildung einer Verbindung der allgmeinen Formel (I),

in der $R_1$ und $R_2$ die oben angegebenen Bedeutungen besitzen und n 3, A eine Methylengruppe und B eine Carbonylgruppe bedeuten,
oder:
ein Morpholin-2,6-dion der allgemeinen Formel (VI)

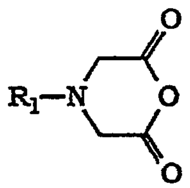

(VI)

in der $R_1$ die oben bezüglich der Formel (I) angegebenen Bedeutungen besitzt, mit einem 4-Piperazinylbutylamin der allgemeinen Formel (VII)

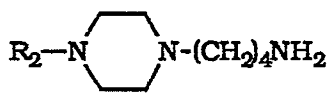

(VII)

in der $R_2$ die bezüglich der Formel (I) angegebenen Bedeutungen besitzt, in einem basischen organischen Lösungsmittel, wie Pyridin, bei einer Temperatur zwischen 40 und 100°C kondensiert zur Bildung einer Verbindung der Formel (I), in der $R_1$ und $R_2$ die oben angegebenen Bedeutungen besitzen und n 4, A eine Methylengruppe und B eine Carbonylgruppe bedeuten,
– oder
ein Piperazin-dion der allgemeinen Formel (VIII)

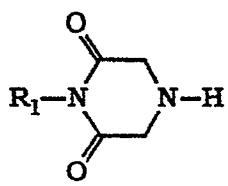

(VIII)

in der $R_1$ die oben bezüglich der Formel (I) angegebenen Bedeutungen besitzt, mit einem Piperazinderivat der allgemeinen Formel (III) in einem polaren organischen Lösungsmittel, wie Butanon-2-, in Gegenwart von anorganischen Salzen, wie Natriumcarbonat und Natriumjodid, bei einer Temperatur zwischen 40 und 100°C kondensiert zur Bildung einer Verbindung der allgemeinen Formel (I), in der $R_1$, $R_2$ und n die oben angegebenen Bedeutungen besitzen und A eine Carbonylgruppe und B eine Methylengruppe bedeuten, und die man anschließend gewünschtenfalls mit einer anorganischen oder organischen pharmazeutisch annehmbaren Säure in ein Additionssalz umwandelt.

13. Pharmazeutische Zubereitung enthaltend als Wirkstoff eine Verbindung nach einem der Ansprüche 1 bis 11 in Kombination oder in Mischung mit einem inerten, nicht toxischen, pharmazeutischen annehmbaren Trägermaterial oder Bindemittel.

14. Pharmazeutische Zubereitung nach Anspruch 13, enthaltend den Wirkstoff in einer Dosis von 0,1 bis 100 mg.

15. Pharmazeutische Zubereitung nach den Ansprüchen 13 und 14 enthaltend als Wirkstoff mindestens eine Verbindung nach einem der Ansprüche 1 bis 11 zur Behandlung von Erkrankungen, die mit anxiolytischen, antiagressiven oder antiopsychotischen Arzneimitteln behandelt werden müssen.

**Patentansprüche für die Vertragsstaaten: AT**

1. Verfahren zur Herstellung der Verbindungen der allgemeinen Formel (I)

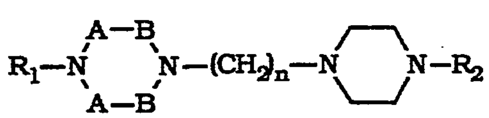

(I)

worin
– A und B jeweils eine Methylengruppe oder eine Carbonylgruppe mit der Maßgabe, daß A und B nicht

gleichzeitig die gleiche Gruppe darstellen,
– $R_1$ eine Diphenylmethylgruppe, die gegebenenfalls an den Benzolringen durch ein Halogenatom, eine Cyclohexylgruppe, eine Pyridylmethylgruppe oder eine gegebenenfalls durch ein Halogenatom oder eine Alkoxygruppe mit 1 bis 4 Kohlenstoffatomen substituierte Benzylgruppe substituiert ist,
– $R_2$ eine Pyrimidinylgruppe oder eine Phenylgruppe, die gegebenenfalls durch ein Halogenatom, eine Alkylgruppe mit 1 bis 4 Kohlenstoffatomen, eine Trifluormethylgruppe oder eine Alkoxygruppe mit 1 bis 4 Kohlenstoffatomen substituiert ist, und
– n eine ganze Zahl mit einem Wert von 2 bis 4 bedeuten, und deren durch Addition einer anorganischen oder organischen pharmazeutisch annehmbaren Säure gebildeten Salze, dadurch gekennzeichnet, daß man
– entweder:
ein Imid der allgemeinen Formel (II)

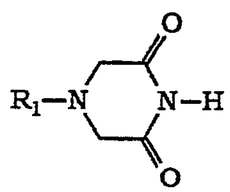 (II)

in der der Substituent $R_1$ die oben angegebenen Bedeutungen besitzt, mit einer Verbindung der allgemeinen Formel (III)

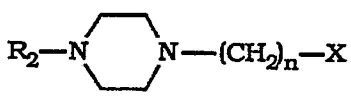 (III)

in der $R_2$ die oben angegebenen Bedeutungen besitzt und n 2 oder 3 und X ein Chloratom oder ein Bromatom bedeuten, in einem wasserfreien, polaren organischen Lösungsmittel bei einer Temperatur zwischen 40 und 100°C und in Gegenwart eines Metallhydrids, wie Natriumhydrid, und eines anorganischen Salzes, wie Natriumjodid, kondensiert zur Bildung der Verbindungen der allgemeinen Formel (I), in der $R_1$ und $R_2$ die oben angegebenen Bedeutungen besitzen und n 2 oder 3, A eine Methylengruppe und B eine Carbonylgruppe bedeuten,
– oder die Verbindung der allgemeinen Formel (II) in einem wasserfreien polaren organischen Lösungsmittel, wie Butanon-2, in Gegenwart eines Metallhydrids, wie Natriumhydrid, mit 1-Brom-3-chlor-propan umsetzt zur Bildung einer Verbindung der allgemeinen Formel (IV)

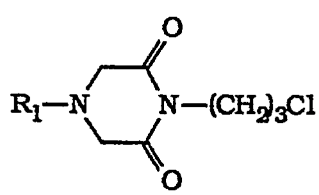 (IV)

in der $R_1$ die oben bezüglich der Formel (I) angegebenen Bedeutungen besitzt, und anschließend das in diese Weise erhaltene 1-(3-Chlor-propyl)-piperazin-2,6-dion mit einem Piperazin der allgemeinen Formel (V)

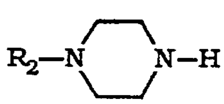 (V)

in der $R_2$ die oben angegebenen Bedeutungen besitzt, in einem polaren organischen Lösungsmittel, wie Butanon-2 in Gegenwart von anorganischen Salzen, wie Natriumcarbonat und Natriumjodid, bei einer Temperatur zwischen 40 und 100°C kondensiert zur Bildung einer Verbindung der allgemeinen Formel (I), in der $R_1$ und $R_2$ die oben angegebenen Bedeutungen besitzen und n 3, A eine Methylengruppe und B eine Carbonylgruppe bedeuten,
oder:
ein Morpholin-2,6-dion der allgemeinen Formel (VI)

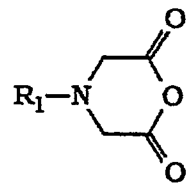

(VI)

in der $R_1$ die oben bezüglich der Formel (I) angegebenen Bedeutungen besitzt, mit einem 4-Piperazinylbutylamin der allgemeinen Formel (VII)

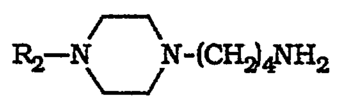

(VII)

in der $R_2$ die bezüglich der Formel (I) angegebenen Bedeutungen besitzt, in einem basischen organischen Lösungsmittel, wie Pyridin, bei einer Temperatur zwischen 40 und 100°C kondensiert zur Bildung einer Verbindung der Formel (I), in der $R_1$ und $R_2$ die oben angegebenen Bedeutungen besitzen und n 4, A eine Methylengruppe und B eine Carbonylgruppe bedeuten,
– oder:
ein Piperazin-dion der allgemeinen Formel (VIII)

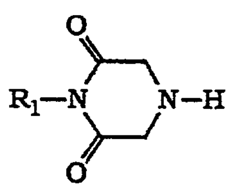

(VIII)

in der $R_1$ die oben bezüglich der Formel (I) angegebenen Bedeutungen besitzt, mit einem Piperazinderivat der allgemeinen Formel (III) in einem polaren organischen Lösungsmittel, wie Butanon-2-in Gegenwart von anorganischen Salzen, wie Natriumcarbonat und Natriumjodid, bei einer Temperatur zwischen 40 und 100°C kondensiert zur Bildung einer Verbindung der allgemeinen Formel (I), in der $R_1$, $R_2$ und n die oben angegebenen Bedeutungen besitzen und A eine Carbonylgruppe und B eine Methylengruppe bedeuten, und die man anschließend gewünschtenfalls mit einer anorganischen oder organischen pharmazeutisch annehmbaren Säure in ein Additionssalz umwandelt.

**Claims for the contracting states: BE, CH, DE, FR, GB, IT, LI, LU, NL, SE**

1. Compounds of general formula I

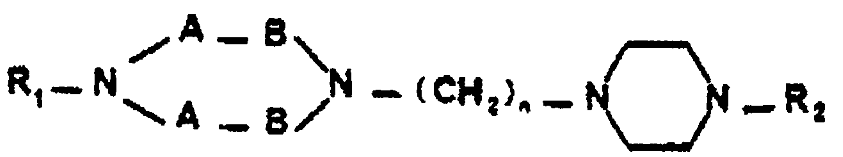

(I)

in which
– A and B each denote a methylene radical or a carbonyl radical with the proviso, however, that A and B never simultaneously denote the same radical,
– $R_1$ denotes a diphenylmethyl radical optionally substituted on the benzene rings with a halogen atom, a cyclohexyl radical, a pyridylmethyl group, or a benzyl radical optionally substituted with a halogen atom or with an alkoxy radical containing from 1 to 4 carbon atoms,
– $R_2$ denotes a pyrimidinyl radical or a phenyl radical optionally substituted with a halogen atom, with an alkyl radical having from 1 to 4 carbon atoms, with a trifluoromethyl radical or with an alkoxy radical containing from 1 to 4 carbon atoms,
– n is an integer which can assume values from 2 to 4, and their salts formed by addition to a pharmaceutically acceptable inorganic or organic acid.

2. 2-(2-Fluorobenzyl)-1-{3-[1-(3-trifluoromethylphenyl)-4-piperazinyl]propyl}-2,6-piperazinedione and its addition salts with a pharmaceutically acceptable inorganic or organic acid.

3. 4-(2-Methoxybenzyl)-1-{3-[1-(3-trifluoromethylphenyl)-4-piperazinyl]propyl}-2,6-piperazinedione

and its addition salts with a pharmaceutically acceptable inorganic or organic acid.

4. 4-(2-Pyridylmethyl)-1-{3-[1-(3-trifluoromethylphenyl)-4-piperazinyl]propyl}-2,6-piperazinedione and its addition salts with a pharmaceutically acceptable inorganic or organic acid.

5. 4-Benzyl-1-{3-[1-(4-fluorophenyl)-4-piperazinyl]-propyl}-2,6-piperazinedione and its addition salts with a pharmaceutically acceptable inorganic or organic acid.

6. 4-(2-Methoxybenzyl)-1-{3-[1,4-fluorophenyl)-4-piperazinyl]propyl}-2,6-piperazinedione and its addition salts with a pharmaceutically acceptable inorganic or organic acid.

7. 4-(2-Methoxybenzyl)-1-{4-[1-(3-trifluoromethylphenyl)-4-piperazinyl]butyl}2,6-piperazinedione and its addition salts with a pharmaceutically acceptable inorganic or organic acid.

8. 4-(2-Pyridylmethyl)-1-{3-[1-(2-chlorophenyl)-4-piperazinyl]propyl}-2,6-piperazinedione and its addition salts with a pharmaceutically acceptable inorganic or organic acid.

9. 4-(2-Pyridylmethyl)-1-{3-[1-(2-methylphenyl)-4-piperazinyl]propyl}-2,6-piperazinedione and its addition salts with a pharmaceutically acceptable inorganic or organic acid.

10. 4-(2-Chlorobenzyl)-1-{3-[1-(3-trifluoromethylphenyl)-4-piperazinyl]propyl}-2,6-piperazinedione and its addition salts with a pharmaceutically acceptable inorganic or organic acid.

11. 1-(3-Methoxybenzyl)-4-{3-[1-(3-trifluoromethylphenyl)-4-piperazinyl]propyl}-1,6-piperazinedione and its addition salts with a pharmaceutically acceptable inorganic or organic acid.

12. Process for preparing the compounds of general formula I, characterized in that:

– either:

an imide of general formula II:

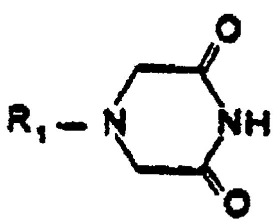

(II)

in which the definition of the substituent $R_1$ remains that mentioned above, is condensed with a compound of general formula III:

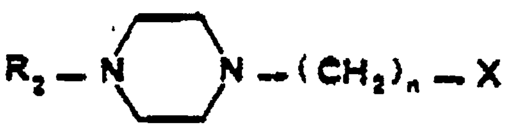

(III)

in which $R_2$ is as defined above, n denotes 2 or 3 and X denotes a chlorine or bromine atom, in a polar anhydrous organic solvent at a temperature of between 40° and 100°C and in the presence of a metal hydride such as sodium hydride and in an inorganic salt such as sodium iodide, to obtain the compounds of formula I in which the definition of $R_1$ and $R_2$ remains that stated above, n equals 2 or 3, A denotes a methylene and B a carbonyl,

or

1-bromo-3-chloropropane is reacted with the compound of general formula II in a polar anhydrous organic solvent such as 2-butanone in the presence of a metal hydride such as sodium hydride, to obtain a compound of general formula IV:

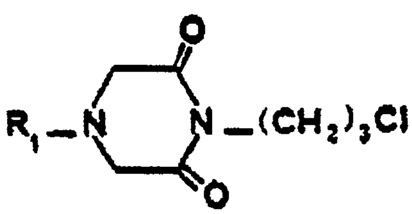

(IV)

in which $R_1$ has the meaning defined above for the formula I, and the 1-(3-chloropropyl)-2,6-piperazinedione thereby obtained is then condensed with a piperazine of general formula V:

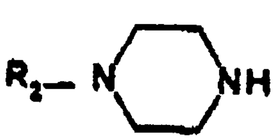

. (V)

in which the definition of $R_2$ remains that stated above, in a polar organic solvent such as 2-butanone in the presence of inorganic salts such as sodium carbonate and sodium iodide and at a temperature of between 40°C and 100°C, to obtain a compound of general formula I in which $R_1$ and $R_2$ have the meanings defined above, n equals 3, A denotes a methylene and B a carbonyl,
or:
a 2,6-morpholinedione of general formula VI:

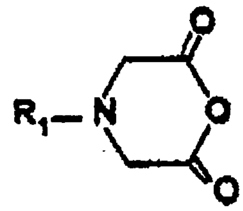

(VI)

in which $R_1$ has the meaning defined above for the formula I, is condensed with a 4-piperazinylbutylamine of general formula VII:

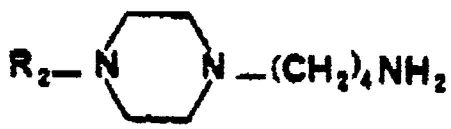

(VII)

in which the definition of $R_2$ remains identical to that given for the formula I, in a basic organic solvent such as pyridine and at a temperature of between 40° and 100°C, to obtain a compound of formula I in which the definition of $R_1$ and $R_2$ remains that stated above, n equals 4, A denotes a methylene and B a carbonyl,
– or:
a piperazinedione of general formula VIII

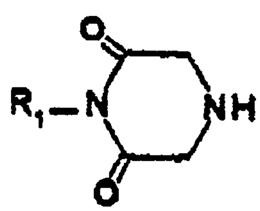

(VIII)

in which $R_1$ has the meaning defined above for the formula I, is condensed with a piperazine derivative of general formula III, in a polar organic solvent such as 2-butanone in the presence of inorganic salts such as sodium carbonate and sodium iodide at a temperature of between 40° and 100°C, to obtain a compound of general formula I in which $R_1$, $R_2$ and n have the meanings defined above, A denotes a carbonyl and B denotes a methylene, and then, if so desired, in that it is converted to an addition salt with a pharmaceutically acceptable organic or inorganic acid.

13. Pharmaceutical compositions containing, as active principle, a compound according to any one of Claims 1 to 11, in combination or mixed with a pharmaceutically acceptable, non-toxic inert excipient or vehicle.

14. Pharmaceutical composition according to Claim 13, containing the active principle at a dose of 0.1 to 100 mg.

15. Pharmaceutical composition according to Claims 13 and 14 containing, as active principle, at least one compound according to Claims 1 to 11, which composition may be used in the treatment of diseases requiring anxiolytic, antiaggression or antipsychotic medication.

**Claims for the contracting states: AT**

1. Process for preparing the compounds of general formula I

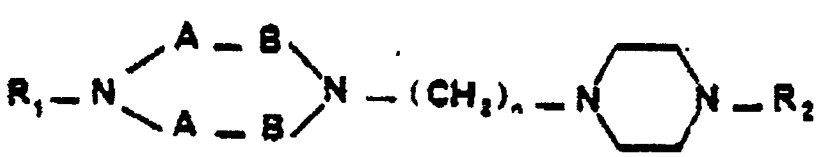

(I)

in which

– A and B each denote a methylene radical or a carbonyl radical with the proviso, however, that A and B never simultaneously denote the same radical,
– $R_1$ denotes a diphenylmethyl radical optionally substituted on the benzene rings with a halogen atom, a cyclohexyl radical, a pyridylmethyl group, or a benzyl radical optionally substituted with a halogen atom or with an alkoxy radical containing from 1 to 4 carbon atoms,
– $R_2$ denotes a pyrimidinyl radical or a phenyl radical optionally substituted with a halogen atom, with an alkyl radical having from 1 to 4 carbon atoms, with a trifluoromethyl radical or with an alkoxy radical containing from 1 to 4 carbon atoms,
– n is an integer which can assume values from 2 to 4, and their salts formed by addition to a pharmaceutically acceptable inorganic or organic acid, characterized in that:
– either:
an imide of general formula II:

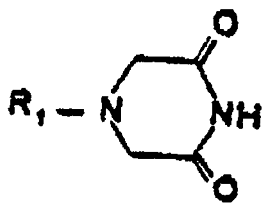

(II)

in which the definition of the substituent $R_1$ remains that mentioned above, is condensed with a compound of general formula III:

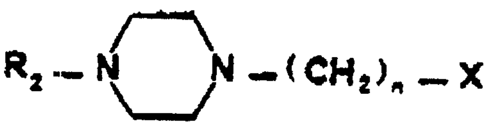

(III)

in which $R_2$ is as defined above, n denotes 2 or 3 and X denotes a chlorine or bromine atom, in a polar anhydrous organic solvent at a temperature of between 40° and 100°C and in the presence of a metal hydride such as sodium hydride and an inorganic salt such as sodium iodide, to obtain the compounds of formula I in which the definition of $R_1$ and $R_2$ remains that stated above, n equals 2 or 3, A denotes a methylene and B a carbonyl,
or
1-bromo-3-chloropropane is reacted with the compound of general formula II in a polar anhydrous organic solvent such as 2-butanone in the presence of a metal hydride such as sodium hydride, to obtain a compound of general formula IV:

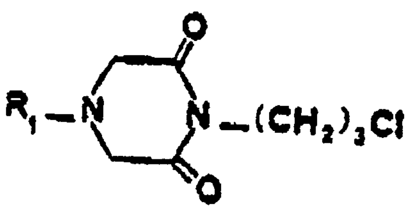

(IV)

in which $R_1$ has the meaning defined above for the formula I, and the 1-(3-chloropropyl)-2,6-piperazinedione thereby obtained is then condensed with a piperazine of general formula V:

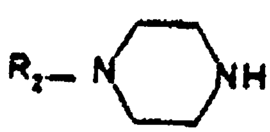

(V)

in which the definition of $R_2$ remains that stated above, in a polar organic solvent such as 2-butanone in the presence of inorganic salts such as sodium carbonate and sodium iodide and at a temperature of between 40°C and 100°C, to obtain a compound of general formula I in which $R_1$ and $R_2$ have the meanings defined above, n equals 3, A denotes a methylene and B a carbonyl,
– or:
a 2,6-morpholinedione of general formula VI:

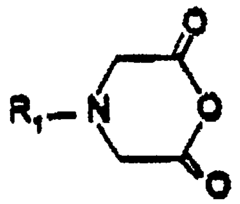

(VI) .

in which $R_1$ has the meaning defined above for the formula I, is condensed with a 4-piperazinylbutylamine of general formula VII:

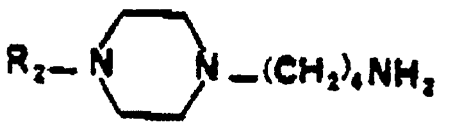

(VII)

in which the definition of $R_2$ remains identical to that given for the formula I, ina basic organic solvent such as pyridine and at a temperature of between 40° and 100°C, to obtain a compound of formula I in which the definitoin of $R_1$ and $R_2$ remains that stated above, n equals 4, A denotes a methylene and B a carbonyl,

– or:

a piperazinedione of general formula VIII

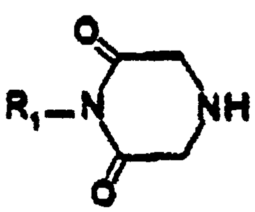

(VIII)

in which $R_1$ has the meaning defined above for the formula I, is condensed with a piperazine derivative of general formula III, in a polar organic solvent such as 2-butanone in the presence of inorganic salts such as sodium carbonate and sodium iodide at a temperature of between 40° and 100°C, to obtain a compound of general formula I in which $R_1$, $R_2$ and n have the meanings defined above, A denotes a carbonyl and B denotes a methylen, and then, if so desired, in that it is converted to an addition salt with a pharmaceutically acceptable organic or inorganic acid.